# EUROPEAN PATENT SPECIFICATION

(11) **EP 2 447 808 B1**
(45) Date of publication and mention of the grant of the patent: **02.09.2015**
(21) Application number: 11008206.2
(22) Date of filing: 11.10.2011
(51) Int. Cl.: G06F 3/01, G09B 21/00, A61F 4/00, G06F 3/00

(54) **Apparatus for operating a computer using thoughts or facial impressions**
Vorrichtung zur Bedienung eines Computers mit Gedanken oder Gesichtsausdrücken
Appareil de fonctionnement d'un ordinateur utilisant des pensées ou impressions faciales

(30) Priority: 18.10.2010 IL 20879610
(43) Date of publication of application: 02.05.2012
(73) Proprietor: Deutsche Telekom AG, 53113 Bonn (DE)
(72) Inventor: Puzis, Rami, Ashdod (IL); Ossmy, Ory, Tel Aviv (IL); Tam, Ofir, Jerusalem (IL); Rozen, Ariel, Tel Aviv (IL); Elovici, Yuval, Arugot (IL)
(74) Representative: Flaccus, Rolf-Dieter

(56) References cited:
- US-A- 4 651 145
- US-A- 5 426 450
- US-A- 6 078 308
- US-A1- 2002 033 803
- US-A1- 2006 125 659

## Description

### Field of the Invention

The present invention relates to Assistive Technology. More particularly, the invention relates to operating a computer using thoughts, emotions or facial impressions.

### Background of the Invention

Disability is the incapacity to normally interact with the outside world. Although it is common to describe the virtual world as approachable by all, in the case of disabled people, this is not always the case. Today, disabled people with hand mobility problems that are simply incapable of operating a mouse or keyboard put their faith in Assistive Technology (TA) and Brain Computer Interface (BCI).

BCI, sometimes called Direct Neural Interface (DMI) or Brain Machine Interface (BMI), is a direct communication pathway between a brain and an external device. BMI enables to record signals from the brain in order to operate a virtual keyboard, control a pointing device to navigate computer cursors on screen, and perform simple tasks by thinking about the task, usually without motor output.

Virtual keyboard is a software component that allows users to enter characters to a computer. A virtual keyboard is usually operated with multiple input devices, which may include an actual mouse. On devices which lack a physical keyboard (such as Personal Digital Assistants (PDA) or touch-screen equipped cell phones), it is common for the user to input text by tapping a virtual keyboard built into the operating system of his device. Virtual keyboards are also used as features of emulation software for systems that have fewer buttons than a computer keyboard have. Virtual keyboards are also providing an alternative input mechanism for users with disabilities who cannot use a physical keyboard.

Pointing device is an input interface that allows a user to input spatial (i.e., continuous and multi-dimensional) data to a computer. Pointing device allows the user to control the computer using physical gestures, namely, point, click, and drag. Movements of the pointing device are echoed on the screen by movements of a cursor and by other visual changes. While the most common pointing device by far is the computer mouse, other pointing devices have been developed. However, "mouse" is commonly used as a metaphor for devices that move the cursor. Several softwares provide full mouse emulation for allowing disabled people to operate a computer.

An emulator in computer sciences is targeted for duplicating the functions supported by a first system using a second system. Utilizing an emulator the second system behaves like the first system. This focus on exact reproduction of external behavior is in contrast to some other forms of computer simulation, which concern an abstract model of the system being simulated. In recent years emulation softwares have been developed for assisting disabled people to operate a computer.

Several solutions for assisting incapable people to operate a computer are offered by different companies. "PointSmart" offers driver enhancement software (http://www.infogrip.com/product view.asp?RecordNumber=988) which makes any mouse, trackball, touchpad, joystick or any other pointing device accessible for users with physical limitations. "SmartClick" (http://www.img-presents.com/smartclk/smc.htm) offers cursor program that provide executing left and right clicks, double click, drag, shift, alt by simply moving a cursor. Another sensory software is "The Grid 2" (http://www.sensorysoftware.com/theg-rid2.html) that provides access to the Windows desktop and other programs, using a built in computer control features. "Dragger" (http://orin.com/access/dragger/index.htm) is a utility for manipulating the left and right mouse buttons of a standard mouse or mouse emulator. Although those solutions assist disabled people to interact with the computer, they require an external mouse or mouse emulator which for many disabled people is not available.

"QualiEYE" suggests cursor control software for people with disabilities (http://qualilife.com/products/index.cfm?id=183&prodType=0&prodTarget= 0). "QuadJoy" (http://www.quadjoy.com/quadjoy.htm) offers controlling a cursor on the screen with the movement of the mouth. "HeadMouseExtreme" (http://orin.com/access/headmouse/index.htm) aims for replacing the standard computer mouse for people who cannot use or have limited use of their hands when controlling a computer or augmentative communication device. However, those systems require the user to move his finger, or head or other organ. It is known that many disabled people suffer a persistent vegetative state, preventing them from moving any of their organs. Utilizing those systems require at least one organ movement, therefore, a completely vegetative user is still incapable of operating a computer using those systems.

Another assistive technology system which addresses disabled people who suffer a persistent vegetative state is the cyberlink-brainfingers (http://www.brainfingers.com/). cyberlink-brainfingers offers hardware and software to control a computer totally hands-free. Cyberlink-brainfingers generates signals by detecting electrical signals from facial muscles, eye movements and alpha/beta waves. A mechanism for mapping the signals into keys is provided. Unfortunately, cyberlink-brainfingers does not operate with new operating systems, and is very expansive.

One revolutionary development in the field of Assistive Technology (AT) is the Emotiv EPOC. Based on the last developments in neuro-technology, Emotiv has developed a revolutionary new personal interface for human computer interaction. The Emotiv EPOC is a high resolution, neuro-signal acquisition and processing wireless neuro-headset. It uses a set of sensors to tune into electric signals produced by the brain to detect user thoughts, emotions and facial impressions, and connects wirelessly to most PCs. However, the software provided with the Emotiv EPOC headset require a training process prior to activation, during this training process the user needs to associate a specific thought to each action he wishes to perform. The headset allows the user to control a cursor using thoughts emotions or facial impressions. For example, if the system is trained to translate a thought regarding football as a right movement, the user is required to think of football five times in order to go on a virtual keyboard from the key "A" to the key "H.

None of the currently available systems provide a satisfying and simple solution to operate a computer by disabled users which are capable only to use their brain. Therefore, there is a need for a system that provides a user interface which enables a user to type on a keyboard and execute operations by using only his thoughts, which incorporates the important benefits of prior art techniques, allows fast operation, and employs a reduced number of thoughts.

It is therefore an object of the present invention to provide a system for controlling a computer or other appropriate electric device, by simply thinking of cognitive thoughts, expressing emotions or moving the face. the signals into keys is provided. Unfortunately, cyberlink-brainfingers does not operate with new operating systems, and is very expansive.

One revolutionary development in the field of Assistive Technology (AT) is the Emotiv EPOC. Based on the last developments in neuro-technology, Emotiv has developed a revolutionary new personal interface for human computer interaction. The Emotiv EPOC is a high resolution, neuro-signal acquisition and processing wireless neuro-headset. It uses a set of sensors to tune into electric signals produced by the brain to detect user thoughts, emotions and facial impressions, and connects wirelessly to most PCs. However, the software provided with the Emotiv EPOC headset require a training process prior to activation, during this training process the user needs to associate a specific thought to each action he wishes to perform. The headset allows the user to control a cursor using thoughts emotions or facial impressions. For example, if the system is trained to translate a thought regarding football as a right movement, the user is required to think of football five times in order to go on a virtual keyboard from the key "A" to the key "H.

US 2006/0125659. discloses a method for inputting text, according to which a bio-signal of a human being is detected and analyzed, so as to extract characteristic information. Then, the characteristic information is converted into an instruction that is used to drive an application program, according to a preset mapping relation that is prepared in accordance with the extracted characteristic information. By performing the instruction, one area of divided areas is selected on a keyboard (e.g., according to a selection area movement instruction) and then, a sub-area in that selected area is selected again, so that finally a letter is selected. Text may also be input by receiving a signal to select one area on a keyboard screen in which letters are arranged in a plurality of divided areas, according to a bio-signal, and then, a signal selecting one sub-area in a plurality of divided sub-areas in the selected area is received, according to a bio-signal. A signal selecting one of letters included in the selected sub-area is received and finally one letter selected and displayed. D1 uses actions to select one group of options out of three groups using hierarchical decomposition for controlling the virtual keyboard. However, D1 is limited to textual inputs via a virtual keyboard, does not present a pointing device and does not suggest or teach using next, select, and cancel operations, which are essential for operating a pointing device, such as a mouse.

US 6,078,308 discloses a method for providing a click surface in a graphical environment implemented on a host computer for use with a force feedback interface device coupled to the host computer. Accordingly, it is determined when a click surface of a first graphical object displayed in a graphical environment is contacted by a user-controlled second graphical object at an original position of the click surface. The usercontrolled second graphical object is displayed at a position that corresponds to a position of a physical user object of the interface device in a physical object workspace. The user object is physically contacted by a user and moveable in a degree of freedom. A force that opposes movement of the user object in a direction that corresponds to movement into the click surface and into the first graphical object is output, while the click surface remains displayed at the original position while the user object moves in the direction corresponding to movement into the click surface. When the user object has moved to or past a trigger position past the original position the click surface, a command gesture signal is sent to the host computer, indicating that the first graphical object has been selected as if a physical input device on the user object had been activated by the user.

US 2002/0033803 discloses an apparatus for interacting with a computer though an input system and controlling a computer display, which comprises an electromagnetic (EM) system including a sensor for producing EM wave, a sensor for receiving EM waves that includes circuits for detecting the electromagnetic waves and for producing signals. The apparatus also includes an antenna operatively connected to the input system and algorithms for converting the signals into relative distance traveled and directs information and into display signals. The apparatus further includes a system for controlling the computer display according to the display signals. The electromagnetic system includes a locator unit and an EM sensor system for sending out a train of EM waves that flood the workspace and measure the relative distance and direction that the locator unit has traveled. The EM sensor system receives reflected EM waves from an antenna in the locator unit, and the sensor produces the distance traveled and direction information.

However, US 6,078,308 and US 2002/0033803 do not use bio-feedback input devices such as EEG or EMG and also, do not employ hierarchical layout for virtual pointing devices or virtual keyboards.

None of the currently available systems provide a satisfying and simple solution to operate a computer by disabled users which are capable only to use their brain. Therefore, there is a need for a system that provides a user interface which enables a user to type on a keyboard and execute operations by using only his thoughts, which incorporates the important benefits of prior art techniques, allows fast operation, and employs a reduced number of thoughts.

It is therefore an object of the present invention to provide a system for controlling a computer or other appropriate electric device, by simply thinking of cognitive thoughts, expressing emotions or moving the face.

Another object of the present invention is to allow disabled people to control a computer using a reduced set of cognitive thoughts, emotions, facial impressions or alternately different actions.

Yet another object of the present invention is to provide a system capable of replacing window's input devices for executing applications and managing the computer's environment.

Still another object of the present invention is to provide a system that learns the user's common behavior and adjust to his needs towards a friendlier and more comfort activation.

Other objects and advantages of the invention will become apparent as the description proceeds.

### Summary of the Invention

Aspects of the invention are disclosed in independent claim 1.The present invention is a computerized system which allows a user to fully control a personal computer or similar electronic device, comprising: (a) a capturer device which captures input from said user, translates said input to actions, and sends said actions to a core engine; (b) a core engine which receives and interprets said actions, said core engine is adapted to change an advanced user interface and the state of said computer or electrical device; and (c) a User Interface which holds the features that defines the screen and interface presented to said user, changing said User Interface changes the screen presented to said user, wherein said User Interface is a virtual interface allowing the handicap or absolutely paralyzed user to fully control a computer or similar electronic device using a small number of actions.

In one embodiment the personal computer or similar electronic device is fully controlled by three identified inputs, said inputs are taken from the group consisting of: head movements, facial movements, thoughts, emotions, and combination thereof.

In one embodiment the capturer device is Emotive EPOC headset.

In one embodiment the core engine is adapted to change the User Interface, as a result said User Interface changes the screen presented to the user and sends the outcomes of said change back to said core engine.

In one embodiment the User Interface is taken from the group consisting of: pointing device, and special keyboard.

In one embodiment the User Interface further provides an initial configuration process for customizing said system in the way most comfortable for each specific user to control the computer or similar electronic device.

### Brief Description of the Drawings

The above and other characteristics and advantages of the invention will be better understood through the following illustrative and non-limitative detailed description of embodiments thereof, with reference to the appended drawings, wherein:
- Fig. 1 schematically illustrates a general framework of the present invention;
- Fig. 2 is a flow diagram of the configuration process;
- Fig. 3 is an exemplary embodiment of the hierarchical pointing device;
- Fig. 4 is an exemplary embodiment of a virtual keyboard hierarchy;
- Fig. 5 is an exemplary block diagram of the virtual keyboard hierarchy; and
- Fig. 6 is an exemplary embodiment of a layout for activating selected applications.

### Detailed Description of the Invention

In the following description, for the purpose of illustration, numerous specific details are provided. As will be apparent to the skilled person, however, the invention is not limited to such specific details and the skilled person will be able to devise alternative arrangements.

The key idea of the present invention is to simulate a pointing device allowing the user to point to a specific location over his screen and click a specific element, or activating a virtual keyboard. The pointing device simulation is targeted for disabled people, and provides the ability to fully activate a computer merely by thoughts, emotions or facial impressions. The present invention allows defining a thinking model that comprises only a few thoughts patterns, three thoughts patterns according to one embodiment. A one time training process associates each thought to a specific action. The system utilizes assistive hardware (e.g., Emotiv EPOC headset) for capturing the user's thoughts. The captured thoughts are translated to actions according to the definition process, according to those actions the system changes the state of an electrical device (e.g., computer, TV, etc) controlled by it.

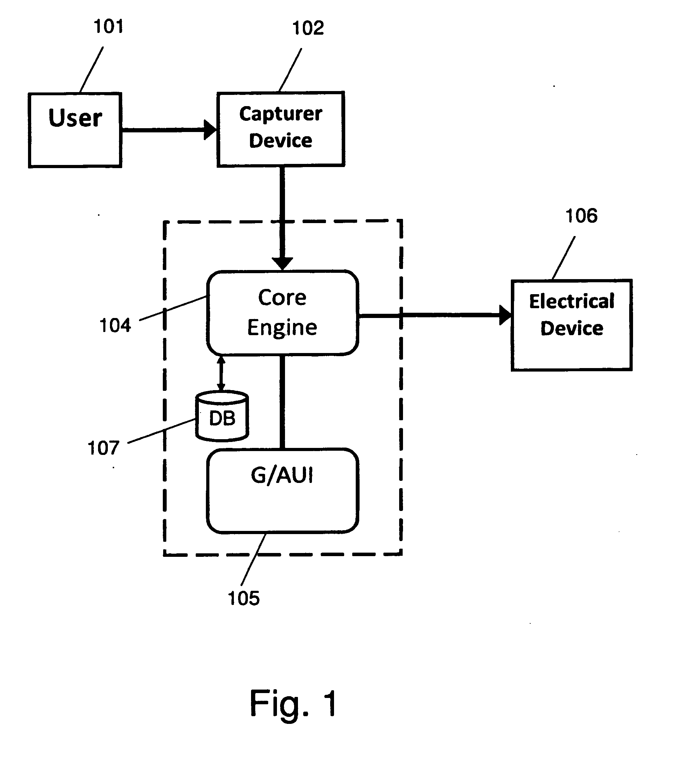

Fig. 1 schematically illustrates a general framework of the present invention. A human user 101 thinks a specific thought or makes a specific action. User's thoughts, emotions or facial impressions are referred hereinafter as user's input. A capturer module 102 captures the user's input. The capturer device 102 is a special assistive hardware which is adapted to capture the user's input and identify it. The capturer module 102 communicates with the system of the present invention 103 and sends a notification regarding the identified input to the system's core engine 104. The system's core engine interprets the user's input and changes an Advanced or Guided User Interface (A/GUI) 105 accordingly. Database (DB) 107 contains a full description of the user's profile and system configuration. During the configuration process, the user can choose a layout of a virtual input device for each application and customize shortcut keys. The database also contains a dictionary for predictive text functionality to improve the interaction of the user with the invention. The database also contains a set of sounds to support audible user interfaces.

The A/GUI 105 holds the features which define the screen and interface presented to the user. Changing the A/GUI causes a change in the screen presented to the user. The changed A/GUI sends in return the outcomes of the captured action to the core engine, namely what is the click or key stroke activated by the translated actions.

After the system's core engine 104 receives from the A/GUI 105 the outcomes (click/key stroke) of the captured action it changes the state of the electrical device 106 (e.g., computer, TV and etc.) connected to it, according to the outcomes of the user's input in the A/GUI. In one embodiment this framework allows the user to control the whole electrical device by three identified inputs. The present invention supports several types of input from the user, e.g., actions only (head movements, facial movements, etc), thoughts only, and combination of both actions and thoughts. The system can be controlled by variety of devices, including but not limited to the EEG headset as mentioned hereinabove. Other devices may be used as input signal providers to MindDesktop including switches, joysticks and trackballs, which are activated by moving a part of the body or even a device measuring sniffing pressure (A. Plotkin, L. Sela, A. Weissbrod, R. Kahana, L. Haviv, Y. Yeshurun, N. Soroker, and N. Sobel. Sniffing enables communication and environmental control for the severely disabled. PNAS, 107(32):14413-14418, 2010).

In one embodiment of the general framework described hereinbefore, the capturer device 102 is EPOC headset. EPOC headset can detect input from the user such as thoughts, emotions and facial impressions. The user's input detected by the EPOC headset transferred as a signal to the system core engine 104 which interprets it to a system action that changes the A/GUI 105 (in this embodiment the A/GUI includes a pointing device that supports click, double click, left click, drag and drop, scroll, and other mouse features. The A/GUI further includes a virtual keyboard that contains letters, numbers, symbols, and special keys that can be used to control applications. For example, play and pause for media player or "send new contacts" for Outlook). Besides changing the A/GUI 105 the system action generated by the system's core engine 104 also transmitted to the electrical device 106. In this embodiment the electrical device is a PC. The system action is transmitted to the PC and changes its state by communicating with its Windows Operating System (OS).

The system of the present invention uses different mechanisms, tools and user interfaces to allow the disabled user to control a computer. The system is very flexible and allows the user to customize these tools and mechanisms to a form that is most comfortable and natural for him. The system provides a unique initial configuration process for customizing the system in the way most comfortable for the disabled person to control the computer.

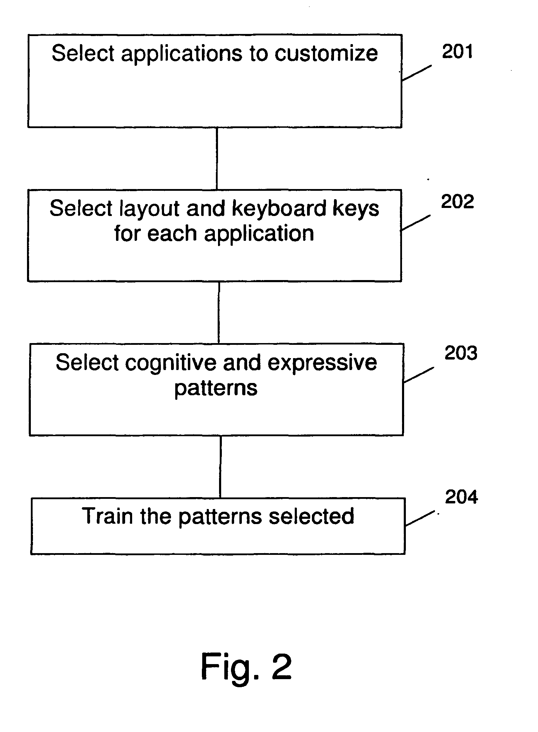

Fig. 2 is a flow diagram of the initial configuration process. Before the disabled user begins controlling a computer with thoughts and/or expressions he chooses his preferences and allows the system to learn his unique brain patterns. The initial configuration process is done by a user which is not disabled, namely can control a regular mouse and keyboard. The entire configuration process is provided to the user through a GUI, wherein tips and explanations are provided to make the configuration process easy and friendly to the average user.

In the first step 201, the user selects the applications he wishes to customize, in one embodiment, the user simply check list all the applications he wishes to customize from a pre-defined list of applications supported by the present invention. The customization allows the user to choose special keys and specific layouts for each application selected. It is important to note the present invention can be used to control any application that can be navigated with a regular mouse or a keyboard.

In the second step 202, the user chooses a layout by which he wishes to control each application selected in step 201 (Windows OS is also an application selected by the user). The present invention offers several layouts which simulate mouse and keyboard use. Additionally, the user can choose which keyboard keys he would like to use for every application selected.

In the next step 203, the user selects which cognitive and expressive patterns will activate which action in the A/GUI. It is important to note that the system is adapted to control computer applications by only a few cognitive and expressive patterns. In this embodiment the computer applications are controlled by exactly three cognitive and expressive patterns. In addition, the user can choose from an array of cognitive and expressive patterns the three which are most suitable for him.

In the final step 204, the user trains the patterns he chose in step 203 so that the system can learn them and react to the brain activity of the user. The training process is required since the patterns selected by the user generate user specific brain signals which should be detected by the system.

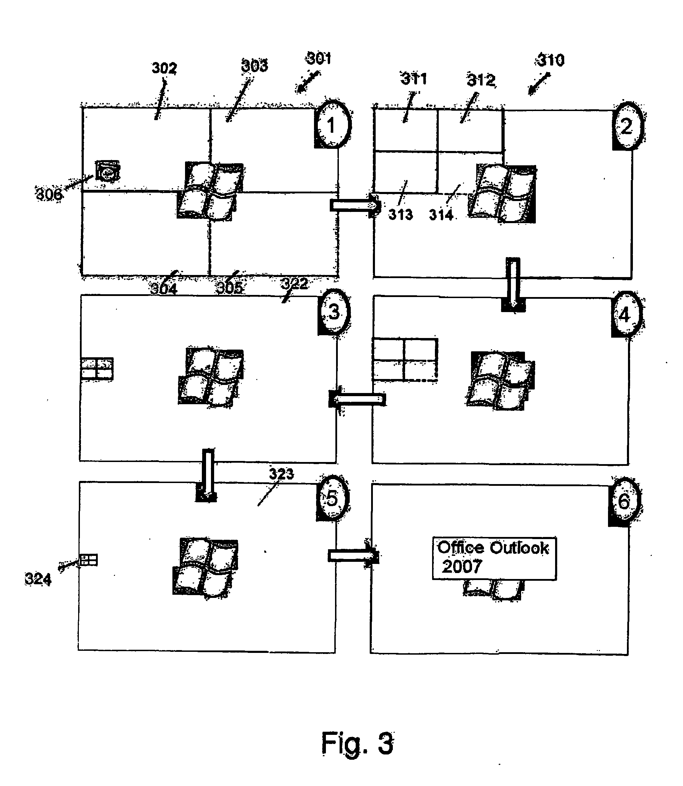

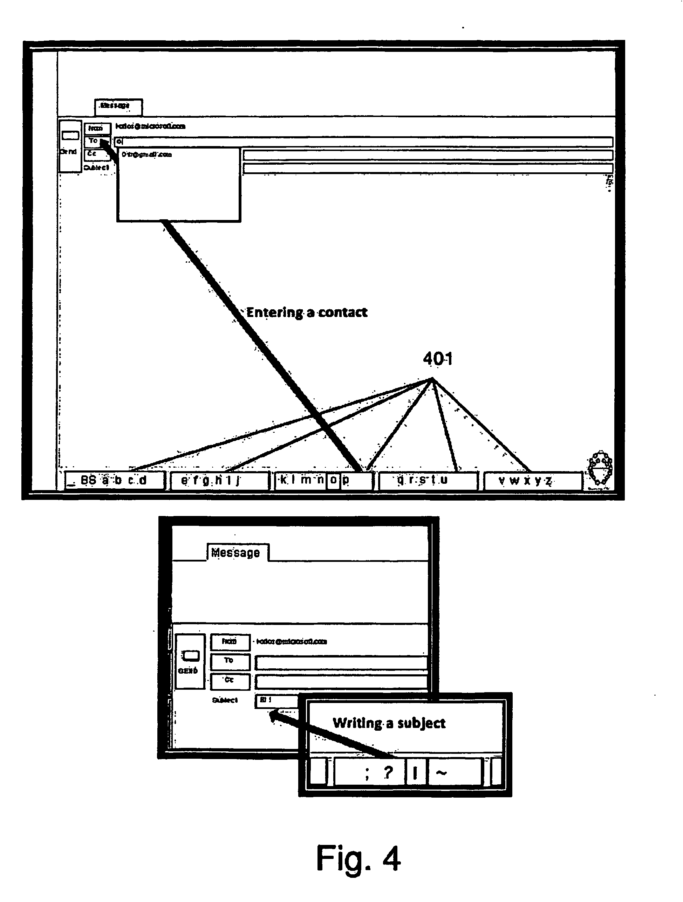

Fig. 3 and Fig. 4 are examples of two virtual input devices: a pointing device and a keyboard respectively. These input devices are controlled by only three user actions. Due to the limitations of EEG/EMG devices the sequence of actions required to complete an interaction with a PC should be as short as possible. In one embodiment the same UI scheme for both types of virtual input devices (keyboard and pointing device) is used.

Fig. 3 is an example of using a hierarchical pointing device layout for Microsoft Outlook activation. The hierarchical pointing device layout simulates a mouse navigation and selection. The hierarchical pointing device layout enables the user to click or double-click on any point on the screen, in the same way as with a regular mouse. The basic idea of this approach is to zoom-in by dividing the screen to four sections having different colors, and selecting one specific section to focus on. The selected section, in turn is divided by colors also to four sections, and one of those four sections is selected, and so on. The user can zoom in a preconfigured number of times, depending on the screen resolution and the desired accuracy of the pointing device. A more accurate device operating on a high resolution screen requires more user actions before the spot of interest can be selected. Finally, at the end of the zooming-in a desired area in the screen is selected. The selection is indicated by a special visual feedback (e.g. icon) or an audible feedback (e.g. beep).

After zooming in to the area of interest, the user can click or double click by performing one or two additional zoom-in actions. After the first zoom-in, the chosen target rectangle blinks. For example, a four seconds blink is provided. If another zoom-in operation is made during these four seconds, the system executes a double click; otherwise, a single click is sent to the operating system.

In general, the pointing device provides the user with basic functionality by using two operations only: scrolling and zooming. However, at each level the user can return to the previous level using the zoom out operation. In this case, the rectangles grow and cover the previous area selected. Zoom out operation is a convenient method for correcting user mistakes such as accidental zoom in or selection of an invalid screen area. It also enables fine navigation of the pointing device when consequent clicks are to be made on different spots in the same screen area. Zooming out can be performed up until the pointing device covers the whole computer screen. From this state, performing additional zoom out operation closes the pointing devices and activates the virtual keyboard.

The virtual keyboard and the pointing device described hereinafter maintain a tree-like hierarchy of internal states in order to make the number of user actions required to complete a task as low as possible. The actual keystrokes and clicks are found at the lowest level of the hierarchy (leafs) while higher levels represent the internal states of an input device. The root of the tree is the entry point to each input device. Users navigate through the hierarchy using three actions, namely scroll, zoom in, and zoom out. Scrolling, changes the virtual device states on the same hierarchy level (i.e. select a sibling). Zooming in, expands the current state allowing the user to scroll through the children. Zooming in on a leaf state communicates the picked input to the operating system. Zooming out, collapses the current state and expands the parent of the hierarchy. Zooming out on a root state is equivalent to canceling the input operation.

As mentioned hereinbefore, the system is adapted to provide the desired control utilizing three actions only. The three actions required for operating the hierarchical pointing device are:
1. Moving across the four sections.
2. Selecting a section to zoom into or clicking on the screen when the zooming reaches the "deepest" level.
3. Zooming out in order to select different section.

In the example shown in Fig. 3 the first computer screen 301 is divided into four sections 302-305. Each section in the screen is indicated by a semi-transparent color cover. If the user wishes to start Microsoft Outlook application he is required to click its Desktop icon 306 which is located in section 302. The user selects to zoom into section 302. In the next screen 310 the screen section previously indicated by 302 is divided into four sections 311-314. The hierarchic zooming continues in screens 321-323 until the color sections 324 cover the Microsoft Outlook icon. In this "deepest" level the icon is clicked utilizing the same input used for the previous zooming.

The present invention allows the user to select different user interfaces and layouts for simulating the use of a pointing device or a real keyboard. One of the supported keyboard layouts is the "Groups keyboard". Fig. 4 is a schematic example of a Groups keyboard 401. The Groups keyboard provides the ability to control which buttons appear on it, and in which order. Moreover, it allows defining extra keys or key combinations which fit to specific applications. The Groups keyboard is a general keyboard that fits most applications which can be used with keyboard and mouse.

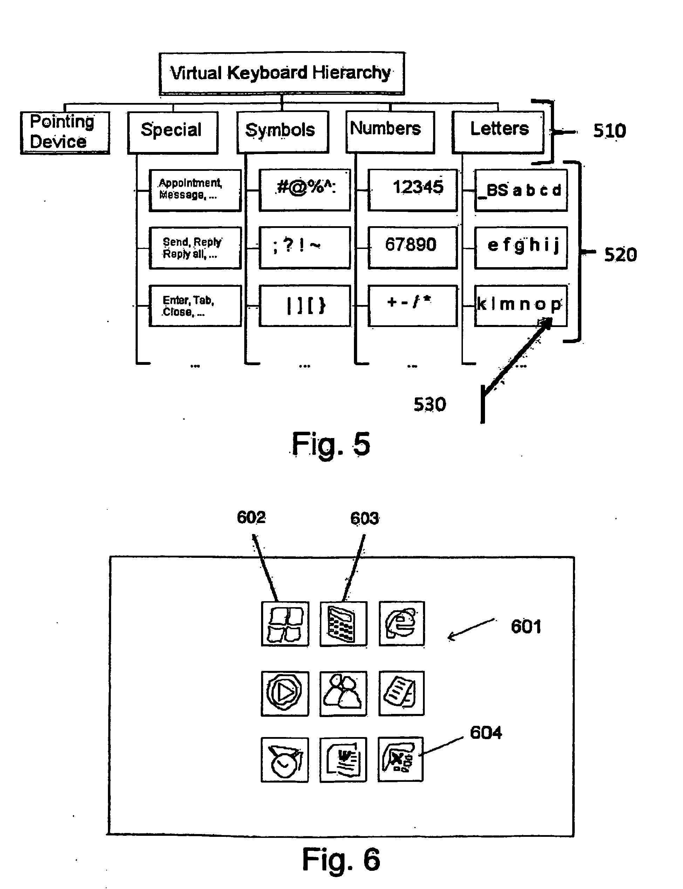

Fig. 5 is an exemplary block diagram of a Groups Keyboard. The Groups Keyboard according to this embodiment contains, three levels. First level 510 in contains:
1. Special keys: shortcut keys and special keys for applications (for example, 'send' key for Mail programs or 'Address bar' key for Web browser programs);
2. Symbols: symbol keys (for example, ',' or '/');
3. Numbers: numeric and arithmetic symbols (for example, '+' or '-');
4. Letters: letter keys, space, and backspace; and
5. Desktop: special input state in the first level that activates the pointing device. It can also be regarded as a "cancel typing" command.

On the second level 520, special keys, symbols, numbers, and letters are expanded to groups of keys. Assuming that the user selects the group 'letters' in the first level 510, the system allows him to scroll through the groups of letters in the second level 520. Zooming in to a selected group of keys shifts the focus to this group (the third level 530 in the hierarchy), and enables the user to scroll through the keys. Once a single key is selected, a "Zoom in" operation on the third level 530 is equivalent to pressing this key on a keyboard. In the case of special keys, an associated command is communicated to the operating system via the MindDesktop core component.

Besides providing different user interfaces of virtual input devices to the user, the present invention also supports the addition of application specific keyboards, which are designed specifically for operating special and common applications. For example, the present invention supports the addition of a special keyboard for Windows media player.

Fig. 6 is one exemplary embodiment of a layout for activating selected applications. Grid Desktop is an alternative layout for activating applications from a pre-defined group 501. The Grid Desktop layout also allows the user to simply navigate the applications icons 502-504 in the pre-defined group 501. The user can select the application he wishes to start by simply clicking on the icon of the desired application.

In order to enhance the user experience with the system of the present invention, a "Designer" tool is provided by the present invention. The "Designer" is a key feature in customizing the system, which enables the user to accomplish several tasks and achieve more natural and flexible controlling. The "Designer" allows the user to create new special application in the system and add special keys to it. When the generated application is activated, a corresponding interface layout, such as the "Groups Keyboard" is assigned to it. The "Designer" also allows the user to add a completely new layout to the system. The added layout can be utilized by different applications. In addition, the "Designer" allows the user to add special keys to an already supported application.

While some embodiments of the invention have been described by way of illustration, it will be apparent that the invention can be carried out with many modifications, variations and adaptations, and with the use of numerous equivalents or alternative solutions that are within the scope of persons skilled in the art, without departing from the invention or exceeding the scope of the claims.

## Claims

1. A computerized system which allows a handicapped or a paralyzed user to fully control a personal computer or similar electronic device, comprising:
a) a capturer device (102) being capable of capturing input taken from the group consisting of: head movements, facial movements, thoughts, emotions, and combination thereof from said user, translating said input to actions, and sending said actions to a core engine;
b) a core engine (104) which receives and interprets said actions, said core engine is adapted to change an advanced user interface and the state of said computer or electrical device; and
c) an advanced User Interface (105) which holds the features that defines the screen and interface presented to said user, said User Interface changes the screen presented to said user;
wherein said user interface includes a hierarchical pointing device layout that simulates a mouse navigation and selection, for allowing said user to click or double-click on any point on said screen and to zoom-in by dividing the screen (301) to sections (302-305) having different colors, and selecting one specific section to focus on, wherein the selected section is divided by colors also to four sections, and one of those four sections is selected, repeatedly.

2. A system according to claim 1, wherein the personal computer or similar electronic device is fully controlled by three identified inputs, said inputs are taken from the group consisting of: head movements, facial movements, thoughts, emotions, and combination thereof.

3. A system according to claim 1, wherein the capturer device (102) is Emotive EPOC headset.

4. A system according to claim 1, wherein the core engine (104) is adapted to change the User Interface (105), as a result said User Interface (105) changes the screen (301) presented to the user and sends the outcomes of said change back to said core engine (104).

5. A system according to claim 1, wherein the User Interface (105) is taken from the group consisting of: pointing device, and special keyboard.

6. A system according to claim 1, wherein said User Interface (105) further provides an initial configuration process for customizing said system in the way most comfortable for each specific user to control the computer or similar electronic device.

## Patentansprüche

1. Computergestütztes System, das einem behinderten oder gelähmten Benutzer die vollständige Steuerung eines Personal-Computers oder eines ähnlichen elektronischen Gerätes ermöglicht, umfassend:
a) eine Erfassungsvorrichtung (102), die in der Lage ist, Eingaben zu erfassen, welche aus der Gruppe bestehend aus Kopfbewegungen, Gesichtsbewegungen, Gedanken, Emotionen des Benutzers und Kombinationen davon ausgewählt sind, diese Eingaben in Aktionen zu übersetzen und diese Aktionen an einen Kernprozessor zu senden,
b) einen Kernprozessor (104), der diese Aktionen empfängt und interpretiert, wobei der Kernprozessor dazu ausgelegt ist, eine erweiterte Benutzerschnittstelle (105) und den Status des Computers oder des elektronischen Gerätes zu ändern, und
c) eine erweiterte Benutzerschnittstelle (105), welche die Merkmale aufweist, die den Bildschirm und die Schnittstelle, die dem Benutzer präsentiert werden, definieren, wobei die Benutzerschnittstelle den Bildschirm, der dem Benutzer präsentiert wird, verändert,
wobei die Benutzerschnittstelle ein hierarchisches Zeigevorrichtungs-Layout beinhaltet, das eine Maus-Navigation und Auswahl per Maus simuliert, welches dazu dient, dem Benutzer das Klicken oder Doppelklicken auf irgendeinen Punkt auf dem Bildschirm sowie das Heranzoomen zu ermöglichen, und zwar durch wiederholtes Aufteilen des Bildschirms (301) in Bereiche (302-305) unterschiedlicher Farbe und Auswählen eines bestimmten Bereichs zur Fokussierung auf diesen Bereich, wobei der ausgewählte Bereich ebenfalls durch Farben in vier Bereiche aufgeteilt und einer dieser vier Bereiche ausgewählt wird.

2. System nach Anspruch 1, bei dem der Personal-Computer oder das ähnliche elektronische Gerät vollständig durch drei identifizierte Eingaben gesteuert wird, wobei die Eingaben aus der Gruppe bestehend aus Kopfbewegungen, Gesichtsbewegungen, Gedanken, Gefühlen und Kombinationen davon, ausgewählt sind.

3. System nach Anspruch 1, bei dem die Erfassungsvorrichtung (102) ein Emotive EPOC-Headset ist.

4. System nach Anspruch 1, bei dem der Kernprozessor (104) dazu ausgelegt ist, die Benutzerschnittstelle (105) zu verändern, als Folge davon verändert die Benutzerschnittstelle (105) den Bildschirm (301), der dem Benutzer präsentiert wird und sendet die Ergebnisse dieser Veränderung zurück an den Kernprozessor (104).

5. System nach Anspruch 1, bei dem die Benutzerschnittstelle (105) aus der Gruppe bestehend aus Zeigevorrichtung und spezieller Tastatur ausgewählt ist.

6. System nach Anspruch 1, bei dem die Benutzerschnittstelle (105) des Weiteren einen initialen Konfigurationsprozess bereitstellt für die Anpassung des Systems an die Kundenwünsche in der für jeden spezifischen Nutzer zur Steuerung des Computers oder des ähnlichen elektronischen Gerätes komfortabelsten Art und Weise.

## Revendications

1. Un système informatisé qui permet à un utilisateur handicapé ou paralysé de commander totalement un ordinateur personnel ou un dispositif électronique similaire, comprenant :
a) un dispositif de capture (102) qui est capable de capturer une entrée prélevée dans le groupe se composant de : mouvements de tête, mouvements du visage, pensées, émotions, et des combinaisons de ceux-ci provenant dudit utilisateur, de convertir ladite entrée en actions et d'envoyer lesdites actions à un moteur central,
b) un moteur central (104) qui reçoit et interprète lesdites actions, ledit moteur central étant adapté de façon à modifier une interface utilisateur avancée et l'état dudit ordinateur ou dispositif électronique, et
c) une interface utilisateur avancée (105) qui contient les caractéristiques qui définissent l'écran et l'interface présentés audit utilisateur, ladite interface utilisateur modifiant l'écran présenté audit utilisateur,
dans lequel ladite interface utilisateur comprend une disposition de dispositif de pointage hiérarchique qui simule une navigation de souris et une sélection, de façon à permettre audit utilisateur de cliquer ou double-cliquer sur tout point sur ledit écran et de zoomer vers l'avant en divisant l'écran (301) en sections (302-305) possédant différentes couleurs, et de sélectionner une section spécifique sur laquelle se concentrer, la section sélectionnée étant divisée par couleurs également en quatre sections et une de ces quatre sections est sélectionnée, de manière répétée.

2. Un système selon la revendication 1, dans lequel l'ordinateur personnel ou le dispositif électronique similaire est totalement commandé par trois entrées identifiées, lesdites entrées étant prélevées dans le groupe se composant de :
mouvements de tête, mouvements du visage, pensées, émotions, et des combinaisons de ceux-ci.

3. Un système selon la revendication 1, dans lequel le dispositif de capture (102) est un casque EPOC émotionnel.

4. Un système selon la revendication 1, dans lequel le moteur central (104) est adapté de façon à modifier l'interface utilisateur (105), en conséquence ladite interface utilisateur (105) modifie l'écran (301) présenté à l'utilisateur et envoie les résultats de ladite modification en retour audit moteur central (104).

5. Un système selon la revendication 1, dans lequel l'interface utilisateur (105) est prélevée dans le groupe se composant de : dispositif de pointage et clavier spécial.

6. Un système selon la revendication 1, dans lequel ladite interface utilisateur (105) fournit en outre un processus de configuration initiale destiné à personnaliser ledit système de la manière la plus confortable pour chaque utilisateur spécifique de façon à commander l'ordinateur ou le dispositif électronique similaire.
